**Europäisches Patentamt**

**European Patent Office**

**Office européen des brevets**

(11) Veröffentlichungsnummer: **0 176 728 B1**

(12) **EUROPÄISCHE PATENTSCHRIFT**

(45) Veröffentlichungstag der Patentschrift:
26.07.89

(51) Int. Cl.⁴: **A 61 F 2/44**

(21) Anmeldenummer: 85110319.2

(22) Anmeldetag: 17.08.85

(54) Bandscheibenendoprothese.

(30) Priorität: 04.09.84 DD 266959
12.02.85 DD 273192
19.07.85 DD 278792
19.07.85 DD 278793

(43) Veröffentlichungstag der Anmeldung:
09.04.86 Patentblatt 86/15

(45) Bekanntmachung des Hinweises auf die Patenterteilung:
26.07.89 Patentblatt 89/30

(84) Benannte Vertragsstaaten:
AT CH FR GB LI NL SE

(56) Entgegenhaltungen:
CH-A- 624 573
DE-A- 2 263 842
DE-A- 3 023 353

(73) Patentinhaber: Humboldt-Universität zu Berlin, Unter den Linden 6, DDR-1086 Berlin (DD)

(72) Erfinder: Büttner-Janz, Karin, Dr. med., Karl-Marx-Allee 78, DDR-1017 Berlin (DD)
Erfinder: Derr, Bernd, Dipl.-Ing., Pirolstrasse 59, DDR-1141 Berlin (DD)
Erfinder: Erkel, Klaus-Peter, Dipl.-Ing., Ludwig-Renn-Allee 19, DDR-8020 Dresden (DD)
Erfinder: Helisch, Hans-Joachim, Dipl.-Jur., Ulmenstrasse 64/66, DDR-1144 Berlin (DD)
Erfinder: Schellnack, Kurt, Doz. Dr. sc. med., Neue Blumenstrasse 20, DDR-1020 Berlin (DD)
Erfinder: Schumann, Roland, Dipl.-Ing., Rothenbacher Strasse 9, DDR-8027 Dresden (DD)

(74) Vertreter: Zipse + Habersack, Kemnatenstrasse 49, D-8000 München 19 (DE)

## Beschreibung

Die Erfindung betrifft eine mehrteilige Endoprothese des Nucleus pulposus, gemäss dem Oberbegriff des Anspruchs 1, welche zwischen Grund- und Deckplatte zweier benachbarter Wirbelkörper einsetzt wird.

Es sind eine Reihe von Vorrichtungen bekannt, die degenerierte, beschädigte oder zerstörte Bandscheiben ersetzen oder zumindest ersetzen sollen. Dabei gibt es Vorrichtungen, wie in der US-PS 4 401 112 beschrieben, die lediglich eine Druckaufnahme sichern, ohne die physiologische Beweglichkeit wieder herzustellen. Bekannt ist, eine pathologisch veränderte Bandscheibe dadurch zu ersetzen, dass der nach operativer Entfernung des Nucleus pulposus entstandene Hohlraum mit Siliconkautschuk ausgefüllt wird, der an Ort und Stelle auspolymerisiert. Die US-PS 4 394 921 beschreibt eine dübelförmige Bandscheibenprothese. Bekannt sind auch mehrteilige Bandscheibenprothesen aus Metall oder Metall-Kunststoff-Paarungen oder aus Kunststoff, die aus je einem Ober- und Unterteil mit druckknopfartigem, diskus- oder kugelförmigem Zwischenstück (DE-PS 3 023 353/DE-OS 2 263 842/CH-PS 624 573/CH-PS 640 131) bestehen. Sonderkonstruktionen stellen die US-PS 4 309 777 und die SU-PS 895 433 dar.

Die bekannten Vorrichtungen haben den Nachteil, die Funktionen eines Nucleus pulposus entweder nicht, nur teilweise oder nur ungenügend zu ersetzen. Das Einarbeiten von Lagerpfannen in die Spongiose der Wirbelkörper hat den Nachteil, dass dafür z.T. langwierige Manipulationen am Patienten erforderlich sind. Ausserdem ist es nicht wünschenswert, dass die im Verhältnis zur Spongiosa relativ harte Aussenschicht der Wirbelkörper zerstört oder angebohrt wird. Infolge der grossen Kontaktlasten, der ungleichmässigen Druckverteilung und/oder der Unnachgiebigkeit der Materialien besteht nicht nur die Gefahr von Nekrosen oder Knochenresorptionen, sondern auch die Möglichkeit einer mechanischen Zerstörung der Wirbelkörper. Weitere Schwierigkeiten ergeben sich aus den anzuwendenden Operationstechniken und aus der Tatsache, dass die erforderliche Sicherheit hinsichtlich Lageveränderungen bei der Anwendung der Bandscheibenendoprothese nicht gegeben ist.

Das Ziel der Erfindung besteht darin, eine möglichst vollwertige Endoprothese des Nucleus pulposus zu schaffen, die eine Distanzhaltung bzw. -wiederherstellung und eine physiologische Beweglichkeit in dem betroffenen Wirbelsäulenabschnitt sichert. Ausserdem muss die Funktionsfähigkeit über einen langen Einsatzzeitraum bei grösstmöglicher Sicherheit gegen Lageveränderungen garantiert werden.

Der Erfindung liegt die Aufgabe zugrunde, eine Endoprothese des Nucleus pulposus zu schaffen, die biologische und biomechanische Toleranz besitzt, formstabil bei Druckaufnahme ist, die ohne aufwendige Bearbeitung der benachbarten Wirbelkörper einsetzbar ist und die eine mechanische Zerstörung der angrenzenden Wirbelkörper ausschliesst.

Diese Aufgabenstellung wird mit den Mitteln gemäss Anspruch 1 gelöst, Weiterbildungen sind in den Unteransprüchen beschrieben. Die Abschlussplatten und das Distanzstück der Bandscheibenendoprothese ermöglichen in ihrem Zusammenwirken eine Neigung der Wirbelkörper in annähernd physiologischen Massen. Das Distanzstück mit teilsphärischer Oberfläche ist linsenförmig ausgeführt, besitzt einen planen Führungsrand und ist aussen mit einer Ringwulst versehen, die ein Abgleiten bzw. Herausrutschen aus den Abschlussplatten verhindert. Die Höhe des Distanzstückes kann entsprechend der Höhe des Zwischenwirbelraumes variiert werden.

Die symmetrischen Abschlussplatten sind in ihrem Mittelteil konkav ausgebildet, besitzen ebenfalls einen planen Führungsrand und sind im Anschluss an eine Kröpfung mit einer Verzahnung versehen. Das Kröpfungsausmass der Abschlussplatten erfolgt entsprechend der sagittalen Wirbelsäulenkrümmung, so dass die ventrodorsale Höhendifferenz des Zwischenwirbelraumes berücksichtigt werden kann. Zur einfachen intraoperativen Orientierung dient eine ventrale Markierung. Der Krümmungsradius der konkaven Ausnehmung entspricht genau dem Krümmerradius des sphärischen Teils des Distanzstückes.

Es ist möglich, die Abschlussplatten an beiden lateralen Seiten mit flächenhaften Verbreiterungen zu versehen, die so gewählt werden, dass sie eine möglichst grosse Fläche der Wirbelkörpergrund- bzw. Wirbelkörperdeckplatte einnehmen. Dadurch erfolgt eine Reduzierung der Druckbelastung pro Flächeneinheit. Das ist besonders wünschenswert für Wirbelkörper mit reduzierter Belastbarkeit.

Zusätzlich oder auch separat kann die Auflagefläche der Abschlussplatten dadurch vergrössert werden, dass der Hohlraum zwischen der Rückseite des konkaven Mittelteils und dem gekröpften Führungsrand durch eine Scheibe aus alloplastischem Material, das auch bioaktiv sein kann, ausgefüllt wird. Die Verwendung von Knochenzement ist ebenfalls denkbar. Es ist auch möglich, die Abschlussplatten kompakt, d.h. ohne Hohlraum an der Rückseite, herzustellen.

Letzlich ist die Verwendung von Kompaktmaterial für die gesamte Abschlussplatte mit lateralen flächenhaften Verbreiterungen möglich. Dazu sind in einer kompakten Abschlussplatte, die der Wirbelkörpergrund- bzw. Wirbelkörperdeckplatte anliegt, das konkave Mittelteil und eine Ringnut zur Aufnahme des Ringwulstes eines Distanzstückes eingearbeitet. Die Abschlussplatten und das Distanzstück werden aus in der Implantattechnik bewährten Werkstoffen gefertigt; beispielsweise bestehen die Abschlussplatten aus nichtrostendem Metall und das Distanzstück aus medizinischem Polyäthylen oder aus Polyurethan mit hoher Zug- und Druckfestigkeit. Eine umgekehrte Materialkombination ist denkbar. Die Verwendung anderer alloplastischer Materialien, die auch bioaktiv sein können, ist ebenfalls möglich. Sowohl die Abschlussplatten als auch das Distanzstück sind an den Berührungsflächen hochglanzpoliert, um den Abrieb zu minimieren (lowfriction-Prinzip).

Der sicheren Implantatverankerung im Zwischenwirbelraum dient eine randständige oder/und flächenhafte Verzahnung an der Unterseite der Abschlussplatten. Es ist möglich, die Verzahnung oder die gesamte Abschlussplattenunterseite bioaktiv zu beschichten. Es ist aber auch möglich, die Abschlussplatten mit Knochenzement zu verankern.

Zur radiologischen Darstellung können sonst nicht sichtbare Kunststoffteile entsprechend markiert werden.

Die Erfindung ermöglicht erstmals einen annähernd vollständigen Ersatz des Nucleus pulposus und garantiert eine physiologische Beweglichkeit in dem betroffenen Wirbelsäulenabschnitt.

Die Erfindung soll nachstehend an Ausführungsbeispielen näher erläutert werden. Die dazugehörigen Zeichnungen zeigen:

Fig. 1a Abschlussplatte
Fig. 1b Abschlussplatte um 180° gedreht
Fig. 2 Distanzstück
Fig. 3 Draufsicht auf eine Abschlussplatte
Fig. 4 Abschlussplatte mit lateralen Verbreiterungen
Fig. 5 Draufsicht auf eine Abschlussplatte mit lateralen Verbreiterungen
Fig. 6 Kompaktabschlussplatte im Schnitt und in Draufsicht.

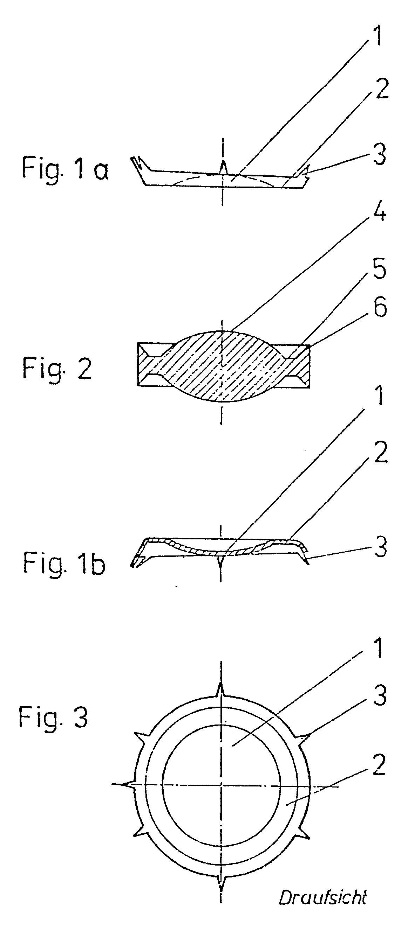

Ausführungsbeispiel 1 (Figuren 1a–3)

Die Abschlussplatten bestehen aus einem nichtrostenden Metall und besitzen ein konkav ausgebildetes Mittelteil 1 und einen ringförmigen, planen Führungsrand 2. Die gekröpften Ränder der Abschlussplatten sind mit einer Verzahnung 3 versehen, die eine sichere Verankerung in den Wirbelkörpern garantieren.

Die Verankerung kann mit einer Randzahnung, bei einer Kompaktvariante mit einer flächenhaften Zahnung, evtl. unter Zuhilfenahme von Knochenzement erfolgen. Die Verzahnung kann auch bioaktiv sein. Zum Erhalt der Lordose der Wirbelsäule ist der vordere Rand der Kröpfung höher als der hintere Rand. Das Distanzstück besitzt ein konvexes Mittelteil 4, das in seinem Krümmungsradius genau dem des konkaven Mittelteils 1 der Abschlussplatten entspricht. Es ist ebenfalls mit einem ringförmigen, planen Führungsrand 5 versehen und besitzt zur Sicherung gegen ein Herausgleiten eine Ringwulst 6. Das Distanzstück kann in seiner Höhe entsprechend den individuellen Bedingungen des Zwischenwirbelraumes gestaltet werden. Es besteht aus einem physiologisch verträglichen Material und ist ebenso wie die Abschlussplatten hochglanzpoliert, um den Abrieb zu minimieren.

Das Distanzstück kann mit einer radiologisch sichtbaren Markierung versehen werden.

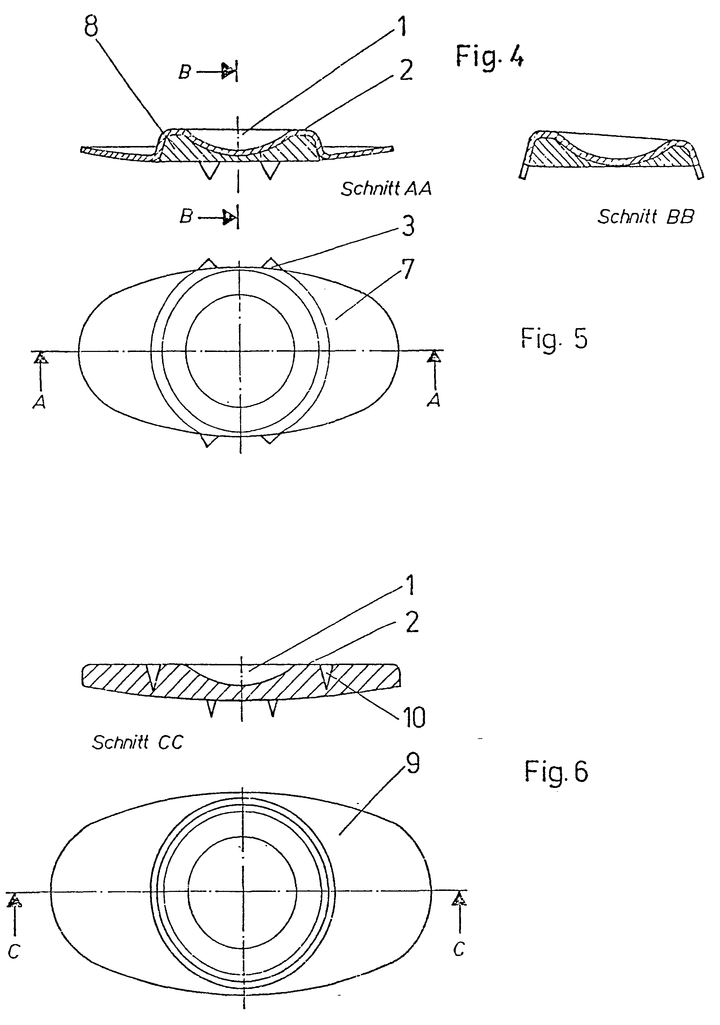

Ausführungsbeispiel 2 (Figuren 4–5)

Die Abschlussplatten sind, wie im Ausführungsbeispiel 1 beschrieben, ausgeführt, jedoch zusätzlich an beiden lateralen Seiten mit flächenhaften Verbreiterungen 7 versehen. Diese flächenhaften Verbreiterungen können der Krümmung der Wirbelkörpergrund- bzw. Wirbelkörperdeckplatte angepasst sein.

Der Hohlraum unter dem planen Führungsrand 2 ist mit einer Scheibe 8 aus alloplastischem Material ausgefüllt. Möglich ist es auch, diesen Hohlraum mit Knochenzement auszufüllen oder von vornherein ein kompaktes Mittelteil zu verwenden.

Hergestellt wird die Abschlussplatte aus einem physiologisch verträglichen Material.

Ausführungsbeispiel 3 (Figur 6)

Die Abschlussplatten für eine mehrteilige Bandscheibenendoprothese werden als sogenannte Kompaktabschlussplatten 9 ausgestaltet. In diese sind das konkave Mittelteil 1 und eine Ringnut 10 zum Eingriff der Ringwulst 6 mit dazwischenliegendem Führungsrand 2 eingearbeitet. Eine Verzahnung 3 ist ebenfalls vorhanden. Die Krümmung der Kompaktabschlussplatte 9 kann ebenfalls der Krümmung der Wirbelkörpergrund- bzw. Wirbelkörperdeckplatte entsprechen.

Patentansprüche

1. Bandscheibenendoprothese, bestehend aus zwei symmetrischen Abschlussplatten mit konkavem Mittelteil und Verankerungsvorsprüngen (3) und mit einem bikonvexen Distanzstück mit gleichem Krümmungsradius wie das konkave Mittelteil, dadurch gekennzeichnet, dass die Abschlussplatten einen ringförmigen, planen Führungsrand (2) aufweisen, der an seiner Aussenkante gekröpft und mit einer randständigen Verzahnung (3) versehen ist, und dass das höhenvariable Distanzstück dazu passend ebenfalls einen ringförmigen, planen Führungsrand (5) besitzt, der von einer Ringwulst (6) umgeben ist, die ein Abgleiten bzw. Herausrutschen aus den Abschlussplatten verhindert.

2. Bandscheibenendoprothese nach Anspruch 1, dadurch gekennzeichnet, dass die Aussenkanten der Abschlussplatten entsprechend der Wirbelsäulenkrümmung unterschiedlich hoch gekröpft sind.

3. Bandscheibenendoprothese nach Anspruch 1 oder 2, dadurch gekennzeichnet, dass der plane Führungsrand (2) der Abschlussplatten mit gegenüberliegenden flächenhaften Verbreiterungen (7) versehen ist, die der Krümmung der Wirbelkörper angepasst sind.

4. Bandscheibenendoprothese nach einem der Ansprüche 1 bis 3, dadurch gekennzeichnet, dass der Hohlraum zwischen Abschlussplatte und Wirbelkörper mit einer Scheibe (8) aus alloplastischem Material und/oder mit Knochenzement ausgefüllt ist.

5. Bandscheibenendoprothese nach einem der Ansprüche 1 bis 3, dadurch gekennzeichnet, dass in einer Kompaktabschlussplatte (9) mit lateralen flächenförmigen Verbreiterungen das konkave Mittelteil (1) und eine Ringnut (10) mit einem dazwischenliegenden Führungsrand (2) eingearbeitet sind.

### Claims

1. Intervertebral disc endoprosthesis comprising two symmetrical end plates having a concave central part and anchoring projections (3) and having a biconvex spacing piece with the same radius of curvature as the concave central part, characterised in that the end plates have a ringshaped, flat guide edge (2) which is bent at its outer edge and is provided with a tooth system (3) continuously round the edge,, and in that the height adjustable spacing piece also has a ringshaped flat guide edge (5) which fits it and which is surrounded by an annular ring (6) with prevents slipping or sliding out from the end plates.

2. Intervertebral disc endoprosthesis according to claim 1, characterised in that the outer edges of the end plates are bent at different heights corresponding to the curvature of the vertebral column.

3. Intervertebral disc endoprosthesis according to claim 1 or 2, characterised in that the flat guide edge (2) of the end plates is provided with opposing two-dimensional extensions (7) which are adapted to the curvature of the spinal column.

4. Intervertebral disc endoprosthesis according to one of claims 1 to 3, characterised in that the chamber between the end plate and spinal column is fillet with a disc (8) made from alloplastic material and/or with bone cement.

5. Intervertebral disc endoprosthesis according to one of claims 1 to 3, characterised in that the concave central part (1) and a ring groove (10) having a guide edge (2) placed between them are incorporated in a compact end plate (9) with lateral two-dimensional extensions.

### Revendications

1. Endoprothèse du disque intervertébral, constituée de deux plaques terminales symétriques présentant une partie médiane concave et des saillies d'ancrage (3) et d'une pièce d'écartement biconvexe de même rayon de courbure que la partie médiane concave, caractérisée en ce que les plaques terminales présentent un bord de guidage annulaire plan (2) qui est rabattu à son arête extérieure et qui est muni d'une denture (3) sur le bord; ert en ce que la pièce d'écartement de hauteur variable, qui s'y adapte, présente également un bord de guidage annulaire plan (5) qui est entouré d'un bourrelet annulaire (6) qui l'empêche de déraper ou de glisser hors des plaques terminales.

2. Endoprothèse du disque intervertébral selon la revendication 1, caractérisée en ce que les arêtes extérieures des plaques terminals sont rabattues sur une hauteur différente selon la courbure de la vertèbre.

3. Endoprothèse du disque intervertébral selon la revendication 1 ou 2, caractérisée en ce que le bord de guidage plan (2) des plaques terminales présente, en surface, des élargissements opposés (7) adaptés à la courbure des vertèbres.

4. Endoprothèse du disque intervertébral selon l'une des revendications 1 à 3, caractérisée en ce que l'espace creux situé entre plaque terminal et vertèbre est rempli d'un disque (8) de matière alloplastique et/ou de ciment osseux.

5. Endoprothèse du disque intervertébral selon l'une des revendications 1 à 3, caractérisée en ce que, dans une plaque terminale compacte (9) présentant en surface des élargissements latéraux, on usine la partie médiane concave (1) et une rainure annulaire (10) avec un bord de guidage (2) situé entre elles.

Fig. 1 a

Fig. 2

Fig. 1b

Fig. 3

Draufsicht

Fig. 4

Schnitt AA

Schnitt BB

Fig. 5

Schnitt CC

Fig. 6

7